# EUROPEAN PATENT APPLICATION

(11) **EP 1 837 053 A1**
(43) Date of publication of application: **26.09.2007**
(21) Application number: 06005853.4
(22) Date of filing: 22.03.2006
(51) Int. Cl.: A61Q 17/04, A61K 8/40, A61K 8/37, A61K 8/97

(54) **Cosmetic composition comprising gingko biloba and sunscreen agents**

(71) Applicant: The Procter and Gamble Company, Cincinnati, Ohio 45202 (US)
(72) Inventor: Gibbons, Clyde, Staines Middlesex TW18 3DE (GB)
(74) Representative: Wilding, Richard Alan

(57) **Abstract**

A cosmetic composition is provided comprising a Gingko biloba extract, an alkyl β,β-diphenylacrylate and/or α-cyano β,β-diphenylacrylate derivative, and a dibenzoyl methane derivative. A cosmetic use of that composition is also provided for preventing sun damage and photo ageing.

## Description

### TECHNICAL FIELD OF THE INVENTION

Cosmetic compositions comprising a Gingko biloba extract, an alkyl β,β-diphenylacrylate and/or α-cyano β,β-diphenylacrylate derivative, and a dibenzoylmethane derivative are provided. Additionally, the use of a Gingko biloba extract in combination with an alkyl β,β-diphenylacrylate and/or α-cyano β,β-diphenylacrylate derivative; and a dibenzoylmethane derivative for preventing sun damage and photo-ageing and/or for treating the skin against sun-damage and photo-ageing is provided. These compositions exhibit a greater sunscreen protection.

### BACKGROUND OF THE INVENTION

It is known that ultraviolet rays (wavelengths from 280nm to 400nm) of sunlight give rise to skin tanning. In particular, it is known that wavelengths from 280mn to 320nm, so-called UVB rays, provoke tanning and erythema commonly associated with photo-ageing. Likewise, it is known that a prolonged and repeated exposure to wavelengths from 320nm to 400nm, so-called UVA rays, also leads to tanning, reduction of the skin elasticity and the appearance of wrinkles leading to a premature skin ageing.

Many active agents exhibiting sunscreen properties have been documented to date. Commonly, the sunscreen effect of these actives and/or compositions is evaluated through the Sun Protection Factor (SPF), which may be measured by *in vitro* and *in vivo* methods. The *in vivo* SPF method consists in exposing a human skin on which a sunscreen composition has been previously applied (versus a skin without sunscreen composition) to ultraviolet radiation from an artificial source and then to determine the ratio between the energy required to induce minimum erythemal response for a protected skin. Many approved *in vivo* methods are known, such as the Colipa method in the European Union (following the International SPF method, 2003) and the FDA SPF method in the USA. In *vitro* methods, such as the Labsphere method using Polymethylmethacrylate hydrophilic plates and the Labsphere 1000S UV Transmittance Analyser are also known.

An SPF of 15 and an SPF of 30 respectfully mean that about 94% and about 97% of the sun rays are blocked. Consequently, the higher the SPF is, the better the protection. To increase the SPF, it is necessary to increase the quantity of sunscreens in an amount that may affect both the aesthetics and the safety of the composition. Therefore, it is important to be able to achieve high SPF without impairing the properties of the composition such as safety, stability, efficacy and application feel once applied onto the skin.

Dibenzoylmethane derivatives, such as butyl methoxydibenzoyl methane, are known hydrophobic organic sunscreens exhibiting a strong absorption of UVA rays. Alkyl β,β-diphenylacrylate and α-cyano β,β-diphenylacrylate derivatives, such as 2-ethylhexyl 2-cyano-3,3-diphenylacrylate, are known hydrophobic organic sunscreens exhibiting a strong absorption of UVB rays. In order to enhance the skin protection against damage caused by a prolonged and repeated exposure to the sun, cosmetic compositions comprising the both UVA and UVB filters may be employed, such as a dibenzoylmethane derivative and an alkyl β,β-diphenylacrylate / α-cyano β,β-diphenylacrylate derivative.

Sunscreen agents only absorb a portion of the UV radiation, the remaining radiations potentially leading to skin damage, In order to prevent and/or treat such damage, cosmetic compositions may further comprise agents exhibiting antioxidant properties. Agents having antioxidant properties may act to mitigate the effects of UV-radiation, for example by regulating the superoxide dismutase activity, or the catalase activity. Numerous antioxidants have been documented to date, such as ascorbic acid, tocopherol, cartenoids.

Gingko Biloba is a large, bold-textured, urban-tolerant shade tree and it is known as a living fossil. That plant is widely used in traditional Chinese medicine and from the late 1950s, its medicinal and cosmetical properties have been studied. Extracts of Gingko Biloba, preferably leaf extracts, are already used in cosmetic compositions. In fact, Gingko biloba is known for preventing and/or treating acne and dermatitis, exhibiting high skin moisture-keeping effect, reducing excess fat, preventing and/or treating scalp conditions, etc.

In order to improve safety, stability and/or efficacy of sunscreen cosmetic compositions, there is a constant need to develop compositions exhibiting strong UV-screening properties without having negative side effects.

Unexpectedly, the inventors have found that a combination of an alkyl β,β-diphenylacrylate and/or α-cyano β,β-diphenylacrylate derivative, a dibenzoylmethane derivative and a Gingko biloba extract exhibits synergistic properties, both in preventing the skin against sun damage and photo-ageing and in treating the skin against sun-damage and photo-ageing .

### SUMMARY OF THE INVENTION

According to a first aspect of the present invention, a cosmetic composition is provided comprising a synergistic combination of a Gingko biloba extract, an alkyl β,β-diphenylacrylate or α-cyano β,β-diphenylacrylate derivative, and a dibenzoylmethane derivative. Furthermore, according to a second aspect of the present invention, the use of the composition according to the first aspect is provided for protecting the skin against sun damage and photo-ageing, preventing such sun damage or photo-ageing and/or treating the skin against sun damage and photo-ageing.

### DEFINITIONS

As used herein, the word "hydrophilic" in relation to the material means that that material is above 10% soluble in water by weight at standard temperature and pressure (STP).

As used herein, the word "hydrophobic" as used in relation to a material means that that material is less than 0.1% soluble in water by weight at standard temperature and pressure (STP).

These and other features, aspects, and advantages of the present invention will become evident to those skilled in the art from a reading of the present disclosure.

### DETAILED DESCRIPTION OF THE INVENTION

Cosmetic compositions according to the present invention comprise a Gingko biloba extract, preferably a hydrophilic extract of Gingko biloba. Any extract of any part of the Gingko biloba plant, preferably which is hydrophilic as defined herein, is suitable for incorporation in the present composition. Preferably, the extract is derived from Gingko biloba leaves. A suitable product containing a hydrophilic extract of Gingko biloba is commercially available under the name "Gingko biloba CG" extract from Cognis (INCI name: Gingko biloba). The cosmetic composition may comprise from 0.01% to 10%, preferably from 0.1% to 5%, and more preferably from 0.75% to 1,5% of a Gingko biloba extract by weight of the composition.

Cosmetic compositions according to the present invention further comprise alkyl β,β-diphenylacrylate and/or α-cyano β,β-diphenylacrylate derivatives. Preferably, α-cyano β,β-diphenylacrylate derivatives comprise ethyl 2-cyano-3,3-diphenylacrylate, 2-ethylhexyl 2-cyano-3,3-diphenylacrylate, or mixture thereof. More preferably, the α-cyano β,β-diphenylacrylate derivative is 2-ethylhexyl 2-cyano-3,3-diphenylacrylate, of which the International Nonproprietary Name is octocrylene. 2-ethylhexyl 2-cyano-3,3-diphenylacrylate is commercially available under the name Parsol 340® from DSM Nutritional Products, Inc. The cosmetic composition may comprise from 0.01 % to 10%, preferably from 0.1 % to 5%, and more preferably from 1 % to 2% of alkyl β,β-diphenylacrylate and/or α-cyano β,β-diphenylacrylate derivatives by weight of the composition.

Cosmetic compositions according to the present invention further comprise dibenzoylmethane derivatives. Preferably, dibenzoylmethane derivatives comprise butyl methoxydibenzoylmethane, ethylhexyl methoxydibenzoylmethane, isopropyl dibenzoylmethane, or mixture thereof. More preferably, the dibenzoyl methane derivative is butyl methoxydibenzoylmethane, of which the International Nonproprietary Name is avobenzone. Butyl methoxydibenzoylmethane is commercially available under the name Parsol 1789® from DSM Nutritional Products, Inc. The cosmetic composition may comprise from 0.01% to 10%, preferably from 0.1 % to 5%, and more preferably from 1% to 3% of dibenzoylmethane derivatives by weight of the composition

In particular, cosmetic compositions according to the present invention comprise a Gingko biloba extract, preferably a hydrophilic extract of Gingko biloba, 2-ethylhexyl 2-cyanow3,3-diphenylacrylate and butyl methoxydibenzoylmethane.

Unexpectedly, the inventors have found that a synergistic effect may be achieved by combining alkyl β,β-diphenylacrylate and/or α-cyano β,β-diphenylacrylate derivatives and dibenzoylmethane derivatives with a hydrophilic extract of Gingko biloba. In particular, the combination of alkyl β,β-diphenylacrylate and/or α-cyano β,β-diphenylacrylate derivatives and dibenzoylmethane derivatives with a hydrophilic extract of Gingko biloba may lead to an unexpected increase of the Sun Protection Factor.

Without wishing to be bound by any theory, it is believed that the hydrophilic extract of Gingko biloba may act as a booster of the sunscreen properties of a combination of alkyl β,β-diphenylacrylate and/or α-cyano β,β-diphenylacrylate derivatives and dibenzoyl methane derivatives. That boosting effect may lead to an unexpected increase of SPF without impairing the properties of sunscreen compositions as might occur if they comprised higher amounts of sunscreen ingredients, such as hydrophobic organic sunscreen agent.

Cosmetic compositions according to the present invention may further comprise at least one additional sunscreen active, i.e. an additional hydrophobic organic sunscreen active, a hydrophilic organic sunscreen active, and/or an inorganic sunscreen. Suitable examples of sunscreens may be found in the CTFA International Cosmetic Ingredient Dictionary and Handbook, 7th edition, volume 2 pp. 1672, edited by Wenninger and McEwen (The Cosmetic, Toiletry, and Fragrance Association, Inc., Washington, D. C., 1997).

Preferred additional hydrophobic organic sunscreen actives according to the invention comprise benzophenone derivatives, such as benzophenone-1 (benzoresorcinol benzoresorcinol), benzophenone-2 (tetraydroxybenzophenone), benzophenone-3 (oxybenzone), benzophenone-4 (sulisobenzone), benzophenone-5 (sulisobenzone sodium), benzophenone-6 (dihydroxy dimethoxy benzophenone), benzophenone-7 (2-benzoyl-4-chlorophenol), benzophenone (8 dioxybenzone), benzophenone-9, benzophenone,10 (mexenone), benzophenone-11, benzophenone-12 (octabenzone); cinnamic derivatives, such as octyl methoxycinnamate (octinoxate), diethanolamine methoxycinnamate; salicylic derivatives, such as ethylhexyl salicylate (octisalate), triethanolamine salicylate, 3,3,5-trimethyleyclohexylsalicylate, homomenthyl salicylate (b.omosaIate); camphor derivatives, such as 4-methylbenzylidene camphor (enzacarnene); triazine derivatives, such hexylethyl triazone; anthranilate derivatives, such as menthyl anthranilate (meradimate); p-aminobenzoic acid derivatives, such as aminobenzoic acid (PABA), glyceryl PABA (lisadimate), octyl dimethyl PABA, ethyl dihydroxypropyl PABA; or mixtures thereof. The cosmetic composition may comprise from 0.01% to 15%, preferably from 0.1% to 10%, and more preferably from 1% to 8% of an additional hydrophobic organic sunscreen by weight of the composition.

Cosmetic compositions according to the present invention may further comprise at least one additional hydrophilic organic sunscreen. Preferably, additional hydrophilic organic sunscreens comprises 2-phenylbenzimidazole-5-sulfonic acid (PBSA), The cosmetic composition according to the invention may comprise from 0.1 % to 5%, preferably from 0.5% to 2.25%, and more preferably from 0.75% to 1.5% of an additional hydrophilic organic sunscreen by weight of the composition.

More preferably, cosmetic compositions according to the present invention comprise a Gingko biloba extract, preferably a hydrophilic extract of Gingko biloba; 2-ethylhexyl 2-cyano-3,3-diphenylacrylate; butyl methoxydibenzoylmethane; and 2-phenylbenzimidazole-5-sulfonic acid.

Cosmetic compositions according to the present invention may comprise at least one inorganic sunscreen active having a mean primary particle size less than to 200 nm, preferably less than 100 nm, and most preferably from 1 to 30 nm. Preferably, inorganic sunscreen actives comprise titanium dioxide, zinc oxide, or mixtures thereof The cosmetic composition according to the invention may comprise from 1% to 15%, preferably from 1% to 10% of an inorganic sunscreen by weight of the composition.

Cosmetic compositions according to the invention may further comprise at least one humectant. Preferably, the humectant comprises polyhydric alcohols. More preferably, the humectant comprises glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof. Still more preferably, the humectant comprises glycerin. The cosmetic composition according to the invention may comprise from 0.1 to 30%, preferably from 5% to 25% and more preferably from 7% to 15% humectant by weight of the cosmetic composition.

Cosmetic compositions according to the present invention may further comprise at least one thickener. Thickeners which may be employed according to the invention comprises C₁₂-C₂₄ fatty acids; C₁₀-C₃₀ fatty alcohols; N-fatty glutamic acid dialylamides; carboxy methyl cellulose or derivatives thereof; polysaccharide gums, such as xanthan gum and guar gum; starch or derivatives thereof; particulate thickeners, such as hydrated colloidal aluminium silicate and magnesium aluminium silicate; carboxyvinyl polymers; sodium acrylate copolymers and hydrophobically modified sodium acrylate copolymers; polyacrylamide copolymers; polyacrylates, such as polyacrylate-13; acrylamide/ammonium acrylate copolymer; ammonium acryloyldimethyl taurate and ammonium acryloyldimethyl taurate crosspolymers and copolymers and hydrophobically modified ammonium acryloyldimethyl taurate copolymers; catinionically charged polymers, such as polyquaternium polymers and polyquaternium copolymers; or mixtures thereof. Preferably, the thickener comprises Luvigel® EM, which is a milky emulsion of sodium acrylates copolymer in caprylic/capric triglyceride and water (INCI name: caprylic/capric triglyceride (and) sodium acrylates copolymer). Luvigel® EM is provided in the form of a water-in-oil emulsion which inverts on addition to water. It is preferred because it may significantly reduce the tack or stickiness when topically applied to the skin. The cosmetic composition according to the invention may comprise from 0.01% to 10%, preferably from 0.1% to 7% and more preferably from 1% to 3% thickener by weight of the composition.

Cosmetic compositions according to the present invention may further comprise at least one additional hydrophilic botanical component. The additional hydrophilic botanical component may comprise any botanical ingredient that fulfils the definition of "hydrophilic" provided herein. The additional hydrophilic botanical component may advantageously comprise an hydrophilic extract of aloe vera leaf (*aloe vera*), argan tree leaf and/or fruit (*argania spinosa*), baobab or Cream of Tartar tree leaf extract (*adansonia digitata*), bearberry leaf (*arctostaphylos*), brahmi (or water hyssop) whole plant (*Bacopa monnieri*), butcher's broom (*ruscus root*), centella asiatica (C*entella asiatica*), chamomile (*Chamomilla Recuita matricaria*), chestnut (*castanea saliva*), clary sage leaf (*salvia sclarea*), eyebright leaf (*euphrasia officinalis*), grape fruit, leaf and/or seed (*vitis vinifera*), green tea leaf (*Camellia Sinensis*), honey (*mel*), horestail leaf (*equisetum arvense*), horse chestnut seed (*aesculus hippocastanum*), Indian cress whole plant (*tropaelolum ajus*), lemongrass plant leaf (*cymbopogon Schoenanthus*), linden wood, leaf and/or bark (*Tilia Cordata*), liquorice root (*glycyrrhiza glabra*), marigold flower (*calendula officinalis*), mimosa leaf and/or bark (*mimosa tenuiflora*), mulberry leaf (*morus nigra*), neem leaf and/or bark (*azadirachta indica*), nettle leaf (*urtica dioica*), oat germ and/or bran (*Avena sativa*), oergano leaf (*Origanum officinalis*), olive leaf and/or fruit (*Olea Europaea*), panax ginseng root (*Panax Ginseng*), plantago leaf (*plantago lanceolata*), propolis (*propolis*), rose hip fruit (*Rosa canina*), rosemary leaf (*Rosmarinus*), sage leaf (*salvia officinalis*), st. Mary's thisle (*silybum marianum*), seabuckthorn, sesame seed (*Sesamum indicum*), sweet manadarin peel (*citrus nobilis*), wheat bran and/or germ (*triticum vulgare*), willow bark (*Salix alba*), witch hazel (*hamamelis Virginia*) or mixtures thereof, preferably an hydrophilic extract of seabuckthorn, rose hip fruit, panax ginseng root or mixtures thereof. The cosmetic composition according to the invention may comprise from 0.0001% to 1%, preferably from 0.001 % to 0.4% and more preferably from 0.002% to 0.15% of an additional hydrophilic botanical component by weight of the composition.

Cosmetic compositions according to the present invention may further comprise at least one additional hydrophobic botanical component. The additional hydrophobic botanical component may comprise any botanical ingredient that fulfils the definition of "hydrophobic" provided herein. The additional hydrophobic botanical component may advantageously comprise argan oil (*Argania Spinosa*), bilberry seed oil (*Vaccinium myrtillus*), blackcurrant seed oil (*Ribes Nigrum*), cotton seed oil (*Gossypium Herbaceum*), cranberry seed oil (*Vaccinium Macrocarpon*), cumin extract (comprising tetrahydrocurcumin), evening primrose oil (*Oenothera Biennis*), grape seed oil (*Vitis Vinifera*), grapefruit seed oil (*Citrus Grandis*), kiwi seed oil (*Actinidia Chinensis*), lavender (*Lavendular Augzistafolia*), ligonberry seed oil (*Vaccinium Vitis-Idaea*), lime seed oil (*Citrus auranifolia*), linseed oil (*Linum usitatissimum*), liquorice root (*glycyrrhiza glabrai*), olive oil (*Olea Europaea*), olive wax (*Olea Europaea*), organic grape seed oil (*Vitis Vinifera*), oriental popy seed oil (*Papaver Orientale*), meadowfoam seed oil (*Limnanthes alba*), neem leaf and/or bark oil (*azadirachta indica*), palm oil (*Elaeis guineenis*), papaya seed oil (*Carica Papaya*), passion fruit seed oil (*Passiflora edulis*), pumpkin seed oil (*Cucrbita pepo*), raspberry seed oil (*Rubus idaeus*), rosehip seed oil (*Rosa Canina*), rosemary oil (*Rosmarinus officinalis*), seabuckthorn seed oil (*Hippophae Rhamnoides*), sesame seed oil (*Sesamum indicum*), shea butter (*butyrospermum parkii*), soy extract, sweet almond oil (*Prunus amygdalus dulcis*), watermelon seed oil (*Citrullus vulgaris*), or mixtures thereof. The cosmetic composition according to the invention may comprise from 0.0001% to 1%, preferably from 0.001% to 0.4%, and more preferably from 0.002% to 0.15% of an additional hydrophobic botanical component by weight of the composition.

Cosmetic compositions according to the invention further comprise at least one hydrophilic vitamin component. The hydrophilic vitamin component may comprise any vitamin or derivative thereof that fulfils the definition of "hydrophilic" provided herein. Preferably, the hydrophilic vitamin component comprises vitamin B₃, salts or esters thereof; pro-vitamin B₅, salts or esters thereof; vitamin C, salts or thereof; hydrophilic derivatives of vitamin E, such as tetramethylchromanol glucosides and 6-hydroxy-2,5,7,8-tetramethylchroman-2-caroxylic acid (commercially available as TROLOX^{™}); or mixtures thereof. Preferably, the hydrophilic vitamin component comprises vitamin B₃, salts or esters thereof; vitamin B₅, salts or esters thereof, or mixtures thereof. More preferably, the hydrophilic vitamin component comprises vitamin B₃, salts or esters thereof, or mixtures thereof. The cosmetic composition according to the invention may comprise from 0.01% to 10%, preferably from 0.1% to 9%, more preferably from 0.5% to 5% of hydrophilic vitamin component by weight of the composition.

Cosmetic compositions according to the present invention may further comprise at least one hydrophobic vitamin component. The hydrophobic vitamin component may comprise any vitamin that fulfils the definition of "hydrophobic" provided herein. The hydrophobic vitamin component may advantageously comprise vitamin E or hydrophobic derivatives thereof, vitamin D or derivatives thereof, or mixtures thereof. The cosmetic composition according to the invention may comprise from. 0.01% to 10%, preferably from 0.1% to 9%, more preferably from 0.5% to 5% of at least one hydrophobic vitamin component by weight of the composition.

Cosmetic compositions according to the present invention may further comprise at least one tanning active. Preferably, said tanning active is selected from dihydroxyacetone (DHA), salts, derivatives or tautomers thereof or mixtures thereof. The cosmetic composition according to the invention may comprise from 0.01% to 10%, preferably from 0.05% to 5%, and more preferably from 0.1 % to 1% of at least one tanning active by weight of the composition.

Cosmetic compositions according to the present invention may further comprise at least one sugar amine. Said sugar amine can be synthetic or natural in origin and can be used as pure compounds or mixtures of compounds (e.g., extracts from natural sources or mixtures of synthetic materials). Preferably, sugar amine comprises glucosamine, N-acetyl glucosamine, mannosamine, N-acetyl mannosamine, galactosamine, N-acetyl galactosamine, isomers thereof (e.g., stereoisomers), salts thereof (e.g., HCl salt), or mixtures thereof. The cosmetic composition according to the invention may comprise from 0.01% to 15%, preferably from 0.1% to 10%, and more preferably from 0.5% to 5% of at least one sugar amine by weight of the composition.

Cosmetic compositions according to the present invention may further comprise at least one hexaminidine compound, salts or derivatives thereof, or mixtures thereof. Preferably, hexaminidine compound comprises compounds corresponding to the following chemical structure: 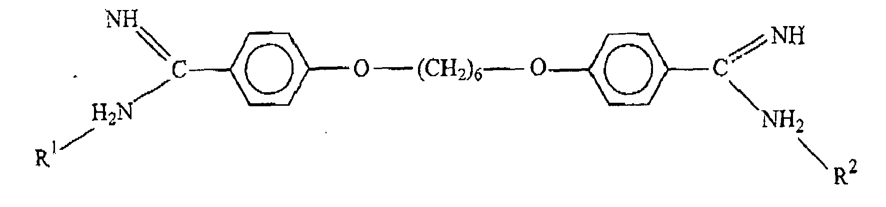 wherein R¹ and R² are organic acids (e.g., sulfonic acids, etc.). The cosmetic composition according to the invention may comprise from 0.001% to 10%, preferably from 0.01% to 5%, and more preferably from 0.02% to 2.5% of a hexaminidine compound by weight of the composition.

The cosmetic composition according to the invention may be in the form of an emulsion, e.g. water-in-oil or oil-in-water emulsion. Advantageously, the cosmetic composition according to the invention is in the form of an oil-in-water emulsion.

The cosmetic composition in the form of an oil-in-water emulsion may further comprise at least one hydrophilic surfactant. Generally, hydrophilic surfactants help disperse and suspend the discontinuous phase within the continuous phase. Preferably, hydrophilic surfactants are selected from nonionic surfactants such as those known in the art. Other suitable surfactants useful herein comprise a wide variety of cationic, anionic, zwitterionic, and amphoteric surfactants such as are known in the art. See, e.g., McCutcheon's, Detergents and Emulsifiers, North American Edition (1986), published by Allured Publishing Corporation. The hydrophilic surfactants useful herein can contain a single surfactant, or any combination of suitable surfactants. The exact surfactant (or surfactants) chosen will depend upon the pH of the composition and the other components present. The cosmetic composition according to the invention may comprise from 0.05% to 10%, preferably from 1% to 6%, and more preferably from 1% to 3% of at least one hydrophilic surfactant by weight of the composition.

The oil phase may comprise at least one oily component such as a natural or synthetic oils selected from mineral, vegetable, and animal oils, fats and waxes, fatty acid esters, fatty alcohols, fatty acids and mixtures thereof. For example, the oil phase may comprise saturated and unsaturated fatty alcohols such as behenyl alcohol, cetyl alcohol and stearyl alcohol and hydrocarbons such as mineral oils or petrolatum.

Cosmetic compositions of the present invention may further comprise at least one emollient material including branched chain hydrocarbons having a weight average molecular weight of from 100 to 15,000, preferably from 100 to 1000. Preferably branched chain hydrocarbons comprise isododecane, isohexadecane, isoeicosane, isooctahexacontane, isohexapentacontahectane, isopentacontaoctactane, petrolanum, isopropyl palmitate, isopropyl isostearate or mixture thereof. The composition according to the invention may comprise from 0.1 % to 15%, preferably from 1% to 7%, more preferably from 3% to 5% of at least one emollient material by weight of the composition.

Cosmetic compositions according to the present invention may further comprise at least one particulate material. Particulate materials may comprise colored and uncolored pigments, interference pigments, inorganic powders, composition powders, optical brightener particles, or mixture thereof, said material being known in the art. These particulate materials do not comprise inorganic sunscreen active. Preferably, particulate materials comprise free-flowing, solid (i.e. not hollow) materials that are insoluble in both water and oil, with a median particle size of from 0,1µm to 75 µm, more preferably from 0,2µm to 30 µm, and with a refractive index of from 1.3 to 1.7, said materials being dispersed in the composition. Suitable particulate materials are organic or organosilicone or inorganic. The composition according to the invention may comprise from 0.01% to 20%, preferably from 0.05% to 10%, and more preferably from 0.1% to 5% particulate materials by weight of the composition.

The oil-in-water emulsion according to the present invention may further comprise a structuring agent to assist in the formation of a liquid crystalline gel network structure. Preferably, structuring agents are selected from stearic acid, palmitic acid, stearyl alcohol, cetyl alcohol, behenyl alcohol, steareth-2, steareth-21, or mixtures thereof. The cosmetic composition according to the invention may comprise from 0.5% to 20%, preferably from 1% to 10%, more preferably from 1% to 5% of a structuring agent by weight of the composition.

A variety of additional optional ingredients may be incorporated into the compositions of the present invention. Non-limiting examples of these additional ingredients include additional skin care actives such as bisabolol, dialkanoyl hydroxyproline compounds, farnesol, flavonoids, guanidine (e.g., amino guanidine), N-acyl amino acid compounds, peptides (e.g. Matrixyl [Pal-KTIKS]), phytantriol, phytosterols, salicylic compounds, urea as well as compounds such as anti-acne compounds (e.g. resorcinol, erythromycin)-, antioxidants compounds (e.g., phytosterols, lipoic acid); skin soothing and healing agents; anti-wrinkle/anti-atrophy actives; conditioning agents; anti-inflammatory agents; skin lightening agents; antimicrobial/antibacterial/antifungal actives; chelators and sequestrants; and agents suitable for aesthetic purposes such as essential oils, fragrances, skin sensates, opacifiers, aromatic compounds (e.g., clove oil, menthol, camphor, eucalyptus oil, and eugenol), preservatives, or mixtures thereof.

The composition of the present invention may be useful for treating a number of mammalian keratinous tissue conditions. Such cosmetic treatment of keratinous conditions can include prophylactic and therapeutic regulation. More specifically, such cosmetic composition can be useful for regulating the skin barrier function, the skin tone/eveness, the skin texture, for providing antioxidant effects, etc.
For instance, the cosmetic composition of the present invention can be for regulating visible and/or tactile discontinuities in mammalian keratinous tissue, including discontinuities in skin texture and color.
Regulating keratinous tissue conditions can involve topically applying to the keratinous tissue a safe and effective amount of the composition of the present invention. The amount of the composition that is applied, the frequency of application, and the period of use will vary widely depending upon the level of components of the given composition and the level of regulation desired, e.g., in view of the level of keratinous tissue damage present or expected to occur.

### Examples

The composition detailed below comprises four different phases of components (cf. table below). Phase A and phase B are heated separately at 75°C under agitation at 150 rpm. Phase B is then added to A and the obtained mixture is mixed at 13,000 rpm (using a high shear mixer such as ultra tarax® to emulsify). Said mixture is then cooled to 50°C and phase C is added. The obtained mixture is cooled to 40°C and phase D and then phase E are added.

| **Ingredient** | **Ph** | **1** | **2** | **3** | **4** | **5** | **6** | **7** | **8** |
|---|---|---|---|---|---|---|---|---|---|
| Deionised water | B | Qs | Qs | Qs | Qs | Qs | Qs | Qs | Qs |
| Glycerin | B | 10.0 | 10.0 | 10.0 | 10.0 | 10.0 | 7.0 | 10.0 | 10.0 |
| Octyl methoxycinnamate | A | | | | 6.00 | | | | 5.00 |
| Octyl Salicylate (Ethylhexyl Salicylate) | A | | 4.00 | 5.00 | | 4.00 | 8.00 | 4.00 | |
| Avobenzone (Butyl methoxydibenzoylmethane) | A | 2.00 | 2.00 | 3.00 | 2.00 | 2.00 | 2.00 | 3.00 | 1.0 |
| Octocrylene | A | 1.00 | 1.00 | 2.00 | 1.00 | 10.0 | 2.00 | 1.00 | 5.00 |
| Phenylbenzimidazole Sulfonic Acid | B | 1.00 | | 2.0 | 1.00 | 1.00 | | 1.00 | |
| Capric/Caprylic Triglycerides and Sodium Acrylates Copolymer | D | 3.00 | 2.50 | 2.00 | 1.80 | 2.25 | 1.50 | 2.25 | 2.00 |
| Xanthan gum | B | | | | | | 0.06 | | |
| Niacinamide | B | 5.00 | 3.50 | 2.00 | 2.00 | 2.00 | 1.00 | 2.00 | 5.00 |
| D-Panthenol | B | 1.25 | 1.00 | 0.75 | 0.50 | 0.50 | 0.25 | 0.50 | 1.25 |
| Tocopheryl Acetate | A | 0.50 | 0.05 | 0.25 | 0.25 | 0.50 | 0.25 | 0.25 | 0.50 |
| Vitamin C | E | 0.00 | 3.00 | 1.00 | 0.00 | 0.00 | 0.00 | 0.00 | 0.00 |
| Magnesium Ascorbyl Phosphate | E | 0,50 | | | 0.50 | 0.25 | 0.00 | 0.00 | 0.50 |
| Sodium Ascorbyl Phosphate | E | | | | | | 0.50 | 0.05 | |
| Dimethicone and Dimethiconol | C | 2,00 | 1.00 | | 2.00 | 2,00 | 2.00 | 2.00 | 1.50 |
| Dimethicone 350 CS | C | | 1.00 | 0.50 | | | | | 0.50 |
| Cyclopentasiloxane & Dimethicone copyol | C | | | 2.00 | | | | | |
| Petrolatum | A | | | | | 0.10 | | | 1.50 |
| Isopropyl Isostearate | A | | 1.50 | 1.33 | 1.33 | | | 1.33 | |
| Isopropyl palmitate | A | 1.00 | | | | 1.40 | 1.00 | | 1.50 |
| Cetyl Alcohol | A | 1.00 | 0.80 | 0.90 | 0.80 | 0.70 | 1.00 | 0.80 | 2.00 |
| Behenyl Alcohol | A | | | | 0.60 | 0.50 | | | 0.40 |
| Stearyl Alcohol 95% | A | 0.75 | 0.45 | 0.60 | 0.40 | 0.90 | 0.50 | 0.55 | 1.00 |
| Stearic Acid | A | 0.10 | 0.10 | 0.10 | 0.10 | 0.10 | 0.10 | 0.10 | 0.30 |
| Sorbitan Stearate | A | 0.60 | 0.70 | 1.00 | 0.90 | 1.30 | 0.50 | 0.90 | |
| Cetearyl Glucoside & Cetearyl Alcohol | A | 0.40 0.10 | 0.10 | 0.20 | 0.30 | 0.05 | 0.50 | 0.10 | |
| Polyethylene | C | 1.00 | 1.00 | 2.00 | 1.50 | 0.20 | | 0.80 | 1.00 |
| Aluminum Starch Octenylsuccinate | C | 0,50 | 1.00 | 2.00 | 0.50 | 0.20 | 1.00 | | |
| CI 77891 titanium dioxide | C | 0.10 | 0.50 | 0.25 | 0.30 | | | | |
| Perfume | E | | | | | | 0.20 | | |
| Disodium EDTA | B | 0.10 | 0.10 | 0.10 | 0.10 | 0.10 | 0.10 | 0.10 | 0.10 |
| PEG-100 Stearate | A | 0.10 | 0.10 | 0.10 | 0.10 | 0.10 | 0.10 | 0.10 | 0.30 |
| Ethylparaben | A | 0.10 | 0.20 | 0.20 | 0.20 | 0.20 | 0.20 | 0.20 | 0.10 |
| Methylparaben | B | 0.20 | | | | | | | 0.20 |
| Propyl Paraben | A | 0.10 | 0.10 | 0.10 | 0.10 | 0.10 | 0.10 | 0.10 | 0.10 |
| Benzyl Alcohol | E | 0.25 | 0.25 | 0.25 | 0.25 | 0.25 | 0.25 | 0.25 | 0.25 |
| Sodium Hydroxide | B | | 0.04 | 0.03 | 0.02 | 0.04 | 0.02 | 0.01 | 0.07 |
| Triethanolamine | B | 0.89 | | | | | | | 0.89 |
| Laureth-7 | A | 0.02 | 0.02 | 0.02 | 0.02 | 0.02 | 0.02 | 0.02 | 0.02 |
| Oleth-3 | A | 0.07 | 0.05 | 0.05 | 0-05 | 0.05 | 0.05 | 0.05 | 0.07 |
| Fruitapone® (Hydrophilic extract Sea Buckthorn 5%) | E | | 1.00 | 0.10 | 0.01 | 0.01 | 0.25 | 0.05 | 0.05 |
| Rose Hip Extract | E | | 0.10 | 0.10 | 0.10 | | | 0.10 | 0.10 |
| Grape Seed extract | E | | | | | | 0.05 | | |
| Ginseng | E | | | 0.05 | | | 0.10 | | |
| Lemon Grass | E | | | | 0.05 | | 0.05 | | |
| Wheat Germ Extract | E | | | | | 0.05 | | | |
| Calendula Extract | E | | 0.05 | | | | | 0.05 | 0.05 |
| Honey | E | | | | | 0.05 | | | |
| Sweet Almond Oil | E | | 0.05 | 0.05 | 0.05 | | | 0.05 | 0.05 |
| Ginkgo biloba hydrophilic extract | E | 1.0 | 5.00 | 0.5 | 0.5 | 2.5 | 3.0 | 10 | 0.1 |
| | | **100** | **100** | **100** | **100** | **100** | **100** | **100** | **100** |

## Claims

1. Cosmetic composition comprising a Gingko biloba extract, an alkyl β,β-diphenylacrylate and/or α-cyano β,β-diphenylacrylate derivative, and a dibenzoyl methane derivative.

2. Composition according to claim 1, comprising a hydrophilic extract of Gingko biloba

3. Composition according to claims 1 or 2, comprising from 0.01% to 10%, preferably from 0.1 % to 5%, and more preferably from 0.75% to 1,5% of a Gingko biloba extract by weight of the composition.

4. Composition according to any one of the preceding claims comprising from 0.01% to 10%, preferably from 0.1% to 5%, and more preferably from 1% to 2% of alkyl β,β-diphenylacrylate and/or α-cyano β,β-diphenylacrylate derivatives by weight of the composition.

5. Composition according to any one of the preceding claims comprising from from 0.01% to 10%, preferably from 0.1% to 5%, and more preferably from 1% to 3% of dibenzoylmethane derivatives by weight of the composition.

6. Composition according to any one of the preceding claims comprising a Gingko biloba extract, preferably a hydrophilic extract of Gingko biloba; 2-ethylhexyl 2-cyano-3,3-diphenylacrylate; and butyl methoxydibenzoylmethane.

7. Composition according to any one of the preceding claims further comprising at least one additional hydrophobic organic sunscreen active, preferably said additional hydrophobic organic sunscreen active comprising benzophenone derivatives, cinnamic derivatives, salicylic derivatives, camphor derivatives, triazine derivatives, p-aminobenzoic acid, or mixture thereof.

8. Composition according to any one of the preceding claims further comprising at least one additional hydrophilic organic sunscreen, preferably 2-phenylbenzimidazole-5-sulfonic acid.

9. Composition according to any one of the preceding claims further comprising at least one additional inorganic sunscreen, preferably that additional inorganic sunscreen comprising titanium dioxide, zinc oxide, or mixtures thereof.

10. Composition according to any one of the preceding claims further comprising at least one humectant, preferably that humectant comprising polyhydric alcohols, more preferably glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof.

11. Composition according to any one of the preceding claims further comprising at least one thickener, preferably comprising C₁₂-C₂₄ fatty acids; C₁₀-C₃₀ fatty alcohols; N-fatty glutamic acid dialylamides; carboxy methyl cellulose or derivatives thereof; polysaccharide gums; starch or derivatives thereof; particulate thickeners; carboxyvinyl polymers; sodium acrylate copolymers and hydrophobically modified sodium acrylate copolymers; polyacrylamide copolymers; polyacrylates; acrylamide/ammonium acrylate copolymer; ammonium acryloyldimethyl taurate and ammonium acryloyldimethyl taurate crosspolymers and copolymers and hydrophobically modified ammonium acryloyldimethyl taurate copolymers; catinionically charged polymers; or mixtures thereof.

12. Composition according to any one of the preceding claims further comprising an additional hydrophilic botanical component, preferably an hydrophilic extract of aloe vera leaf, argan tree leaf and/or fruit, baobab or Cream of Tartar tree leaf extract, bearberry leaf, brahmi whole plant, butcher's broom, centella asiatica, chamomile, chestnut, clary sage leaf, eyebright leaf, grape fruit, leaf and/or seed, green tea leaf, honey, horestail leaf, horse chestnut seed, Indian cress whole plant, lemongrass plant leaf, linden wood, leaf and/or bark, liquorice root, marigold flower, mimosa leaf and/or bark, mulberry leaf, neem leaf and/or bark, nettle leaf, oat germ and/or bran, oergano leaf, olive leaf and/or fruit, panax ginseng root extract, plantago leaf, propolis, rose hip fruit, rosemary leaf, sage leaf, st. Mary's thisle, seabuckthorn, sesame seed, sweet manadarin peel, wheat bran and/or germ, willow bark, witch hazel, or mixtures thereof, more preferably an hydrophilic extract of seabuckthorn, rose hip fruit, panax ginseng root or mixtures thereof.

13. Composition according to any one of the preceding claims further comprising a hydrophobic botanical component, preferably comprising argan oil, bilberry seed oil, blackcurrant seed oil, cotton seed oil, cranberry seed oil, cumin extract, evening primrose oil, grape seed oil, grapefruit seed oil, kiwi seed oil, lavender, ligonberry seed oil, lime seed oil, linseed oil, liquorice root, olive oil), olive wax, organic grape seed oil, oriental popy seed oil, meadowfoam seed oil, neem leaf and/or bark oil, palm oil, papaya seed oil, passion fruit seed oil, pumpkin seed oil, raspberry seed oil, rosehip seed oil, rosemary oil, seabuckthorn seed oil, sesame seed oil, shea butter, soy extract, sweet almond oil, watermelon seed oil, or mixtures thereof.

14. Composition according to any one of the preceding claims further comprising at least one hydrophilic vitamin component, preferably comprising vitamin B₃, salts or esters thereof; pro-vitamin B₅, salts or esters thereof; vitamin C, salts or esters thereof; hydrophilic derivatives of vitamin E; or mixtures thereof; more preferably vitamin B₃, salts or esters thereof; vitamin B₅, salts or esters thereof, or mixtures thereof.

15. Composition according to any one of the preceding claims further comprising at least one hydrophobic vitamin component, preferably comprising vitamin E or hydrophobic derivatives thereof, vitamin D or derivatives thereof, or mixtures thereof

16. Composition according to my one of the preceding claims further comprising at least one tanning active, preferably dihydroxyacetone, salts, derivatives or tautomers thereof, or mixtures thereof.

17. Composition to any one of the preceding claims further comprising at least one sugar amine, preferably glucosamine, N-acetyl glucosamine, mannosamine, N-acetyl mannosamine, galactosamine, N-acetyl galactosamine, isomers thereof, salts thereof, or mixtures thereof.

18. Composition to any one of the preceding claims further comprising at least one hexaminidine compound, salts or derivatives thereof, or mixtures thereof

19. Cosmetic composition according to any one of the preceding claims, which is in the form of an emulsion, preferably an oil-in-water emulsion.

20. Cosmetic use of a composition according to claims 1 to 19 for preventing sun damage and photo ageing.
